# EUROPÄISCHE PATENTSCHRIFT

(11) **EP 0 557 818 B1**
(45) Veröffentlichungstag und Bekanntmachung des Hinweises auf die Patenterteilung: **28.08.1996**
(21) Anmeldenummer: 93102216.4
(22) Anmeldetag: 12.02.1993
(51) Int. Cl.: C07D 295/08

(54) **Verfahren zur Herstellung von N-(2-Hydroxypropyl)-2,6-dimethylmorpholin**
Process for the preparation of N-(2-hydroxypropyl)-2,6-dimethylmorpholin
Procédé pour la préparation de N-(2-hydroxypropyl)-2,6-diméthylmorpholine

(30) Priorität: 28.02.1992 DE 4206171
(43) Veröffentlichungstag der Anmeldung: 01.09.1993
(73) Patentinhaber: BASF Aktiengesellschaft, 67063 Ludwigshafen (DE)
(72) Erfinder: Pinkos, Rolf, Dr., W-6702 Bad Duerkheim (DE); Merkle, Hans Rupert, Dr., W-6700 Ludwigshafen (DE); Fischer, Rolf, Dr., W-6900 Heidelberg (DE)

(56) Entgegenhaltungen:
- CS-A- 163 802
- DD-A- 287 192

## Beschreibung

Die Erfindung betrifft ein Verfahren zur Herstellung von N-(2-Hydroxypropyl)-2,6-dimethylmorpholin aus Tris-(2-hydroxypropyl)-amin in Gegenwart von Heterogenkatalysatoren.

Aus DD-A-287 192 ist bekannt, daß Tris-(2-hydroxypropyl)-amin beim Behandeln mit 70%-iger Schwefelsäure unter Wasserabspaltung zu N-(2-Hydroxypropyl)-2,6-dimethylmorpholin cyclisiert. Dabei fällt das Produkt in protonierter Form an und muß durch Behandeln mit Natronlauge freigesetzt werden. Es resultiert wäßrige Natriumsulfatlösung, die aufwendig entsorgt werden muß. Aus dem Beispiel 1 a ergibt sich, daß pro kg N-(2-Hydroxypropyl)-2,6-dimethylmorpholin über 4 kg Natriumsulfat entstehen.

Der vorliegenden Erfindung lag daher die Aufgabe zugrunde, den zuvor genannten Nachteilen abzuhelfen.

Demgemäß wurde ein neues und verbessertes Verfahren zur Herstellung von N-(2-Hydroxypropyl)-2,6-dimethylmorpholin gefunden, welches dadurch gekennzeichnet ist, daß man Tris-(2-hydroxypropyl)-amin in Gegenwart
a) von sauren Katalysatoren oder
b) von Hydrierkatalysatoren in Gegenwart von Wasserstoff cyclisiert.

Das erfindungsgemäße Verfahren läßt sich wie folgt durchführen:
a) Die Cyclisierung kann durch Kontakt von Tris-(2-hydroxypropyl)-amin mit sauren Katalysatoren bei Temperaturen von 100 bis 400°C, bevorzugt 150 bis 350°C, besonders bevorzugt 180 bis 290°C und Drücken von 0,001 bis 50 bar, bevorzugt 0,01 bis 2 bar, besonders bevorzugt 0,1 bis 1 bar vorgenommen werden.
   Die saure Cyclisierung kann kontinuierlich, diskontinuierlich oder absatzweise durchgeführt werden. Bevorzugt arbeitet man in Wirbelbett- oder Festbettreaktoren, wobei in der Flüssigphase oder bevorzugt in der Gasphase cyclisiert werden kann.
   Weiterhin bevorzugt ist die Cyclisierung in einer den Katalysator enthaltenen Vorlage in der Flüssigphase, wobei das Wertprodukt abdestilliert werden kann. Das Tris-(2-hydroxypropyl)-amin kann kontinuierlich oder absatzweise zugegeben werden.
   Die Verweilzeit kann bei den Reaktionen in der Gasphase z.B. 1 bis 5 Sekunden betragen, bei Reaktionen in der Flüssigphase 5 Minuten bis 2 Stunden.
   Als saure Katalysatoren eignen sich beispielsweise Oxide der III. oder IV. Nebengruppe oder der II. bis VI. Hauptgruppe des Periodensystems der Elemente oder Kombinationen derselben wie saure oder supersaure Metalloxide der Nebengruppenelemente z.B. TiO₂, ZrO₂, Fe₂O₃ und ZnO oder z.B. MgO, B₂O₃, Al₂O₃, SiO₂ und SnO₂ oder Kombinationen derselben, wie z.B. TiO₂*ZnO oder Al₂O₃*MgO. Zur Erhöhung der Säurestärke können die Oxide mit Säuren wie z.B. Schwefelsäure oder Phosphorsäure behandelt werden.
   Als saure Katalysatoren eignen sich auch Zeolithe, beispielsweise Vertreter aus der Mordenit-Gruppe oder engporige Zeolithe vom Erionit- bzw. Chabasit-Typ oder Zeolithe vom Faujasit-Typ, z. B. Y-, X- oder L-Zeolithe sowie die sogenannten "ultrastabilen" Zeolithe des Faujasittyps, d.h. dealuminierte Zeolithe.
   Geeignet sind auch Zeolithe mit Pentasilstruktur wie ZSM-5, ZSM-11 und ZBM-10. Diese haben als Grundbaustein einen aus SiO₂-Tetraedern aufgebauten Fünfring gemeinsam. Sie sind durch ein hohes SiO₂/Al₂O₃-Verhältnis gekennzeichnet sowie durch Porengrößen, die zwischen denen der Zeolithe vom Typ A und denen vom Typ X oder Y liegen.
   Ebenso geeignet sind auch Schichtsilikate wie z. B. Tonsil, die mit Aluminium dotiert sein können oder Alumosilikate.
   Ferner sind Heteropolysäuren geeignet. Dies sind anorganische Polysäuren, die im Gegensatz zu Isopolysäuren mindestens zwei verschiedene Zentralatome besitzen. Als Beispiele seien Dodecawolframphosphorsäure H₃PW₁₂O₄₀*xH₂O, Dodecamolybdophosphorsäure H₃PMo₁₂O₄₀*xH₂O genannt. Prinzipiell können die in EP-A-158 229 genannten Katalysatoren und Katalysatorkombinationen eingesetzt werden.
   Bevorzugte Heteropolysäuren sind Heteropolysäuren von Molybdän oder Wolfram mit Phosphorsäure, Tellursäure, Selensäure, Arsensäure, Kieselsäure, insbesondere mit Phosphorsäure.
   Die Protonen der Heteropolysäuren können teilweise durch Metallionen ersetzt sein, bevorzugt sind dabei die Alkali- und Erdalkalimetallionen.
   Geeignet sind auch Phosphate, Hydrogenphosphate und Polyphosphate, die Alkali- oder Erdalkaliionen enthalten können.
   Grundsätzlich sind Kombinationen aus den genannten Katalysatoren geeignet, z. B. Kombinationen aus Aluminiumoxiden, die durch Säuren wie Schwefel- oder Phosphorsäure modifiziert wurden und Heteropolysäuren.
b) Die Cyclisierung von Tris-(2-hydroxypropyl)-amin kann an Hydrierkontakten bei Temperaturen von 150 bis 350°C, bevorzugt 180 bis 310°C und bei Drücken von 1 bis 300 bar, bevorzugt 3 bis 100 bar, besonders bevorzugt 4 bis 50 bar in der Gasphase oder bevorzugt in der Flüssigphase durchgeführt werden. Die Reaktion kann ohne Zugabe von Wasserstoff erfolgen, bevorzugt wird aber in Gegenwart von Wasserstoff gearbeitet. Dabei wird der Reaktionsdruck im wesentlichen durch den Wasserstoffdruck beim Aufpressen und der Druckänderung beim Aufheizen in geschlossenen Druckapparaturen bestimmt.
   Die Durchführung kann diskontinuierlich, z.B. in Autoklaven, oder vorzugsweise kontinuierlich z.B. in Rohrreaktoren durchgeführt werden.
   Als Katalysatoren kommen alle in Frage, die hydrierend wirken, z.B. solche, die Ketone oder Aldehyde katalytisch mit Wasserstoff zu Alkoholen zu hydrieren vermögen.
   Beispiele hierfür sind in Houben-Weyl, Methoden der organischen Chemie, Band IV/1c, Georg Thieme Verlag Stuttgart, 1980, Seite 16-28, 68, 189-224 beschrieben. Es kommen als Hydrierkatalysatoren z.B. Elemente der I. und VI. bis VIII. Nebengruppe des Periodensystems der Elemente z.B. in Form der Metalle, ihrer Oxide und Sulfide in Frage. Sie können z.B. als Trägerkatalysatoren, Skelett- katalysatoren, Schwarzkatalysatoren oder metallische Mischkatalysatoren eingesetzt werden. Beispiele sind Pt-Schwarz, Pt/C, Pt/Al₂O₃, PtO₂, Pd-Schwarz, Pd/C, Pd/Al₂O₃, Pd/SiO₂, Pd/CaCO₃, Pd/BaSO₄, Rh/C, Rh/Al₂O₃, Ru/SiO₂, Ni/SiO₂, Raney-Nickel, Co/SiO₂, Co/Al₂O₃, Raney-Kobalt, Fe, Fe-haltige Mischkatalysatoren, Re-Schwarz, Raney-Rhenium, Cu/SiO₂, Cu/Al₂O₃, Raney-Kupfer, Cu/C, PtO₂/Rh₂O₃, Pt/Pd/C, CuCr₂O₄, BaCr₂O₄, Ni/Cr₂O₃/Al₂O₃, Re₂O₇, Re₂O₇, CoS, NiS, MoS₃, Cu/SiO₂/MoO₃/Al₂O₃ oder Kombinationen aus diesen.
   Bevorzugte Katalysatoren sind solche, die Kupfer als Komponente z.B. in Mengen von 2 bis 90 Gew.-%, bevorzugt 10 bis 80 Gew.-%, enthalten.
   Die Katalysatoren müssen vor der Reaktion nicht notwendigerweise aktiviert werden.
   Die Verweilzeit beträgt in der Regel zwischen 5 Minuten und 3 Stunden.

Bei den Varianten a) und b) entstehen Isomerengemische aus cis- und trans-N-(2-Hydroxypropyl)-2,6-dimethylmorpholin im Verhältnis von ca. 3 : 1.

Es ist überraschend, daß die verbleibende Hydroxylgruppe des N-(2-Hydroxypropyl)-2,6-dimethylmorpholins unter den erfindungsgemäßen Bedingungen weitgehend erhalten bleibt, da aus Houben-Weyl, Band V/1b, Georg Thieme Verlag Stuttgart, 1972, Seite 45 bis 83 bekannt ist, daß sekundäre Hydroxylgruppen bei Temperaturen ab 200°C in Gegenwart der erfindungsgemäßen Katalysatoren bereits Olefin und Wasser bilden.

N-(2-Hydroxypropyl)-2,6-dimethylmorpholin kann z. B. als Ausgangsstoff für Fungizide zur Bekämpfung phytopathogener Pilze (DD-A-287 195) verwendet werden.

### Beispiele

### Beispiel 1

In einem Wirbelofen (300 ml Volumen) wurde in einem heißen Stickstoffstrom (60 l/h) Tris-(2-hydroxypropyl)-amin (20 g/h, 90%-ige Reinheit) verdampft (1013 mbar) und bei 220°C an einem Katalysatorgemisch aus 80 g eines mit Phosphorsäure (5 Gew.-%) dotierten Aluminiumoxids und 40 g getrockneter Molybdatophosphorsäure 8 Stunden lang umgesetzt. Der dunkelgefärbte Reaktionsaustrag bestand aus zwei Phasen. Die untere Phase bestand vorwiegend aus Reaktionswasser, die obere, organische Phase enthielt nach gaschromatographischer Analyse 77 Gew.-% N-(2-Hydroxypropyl)-2,6-dimethylmorpholin neben 3 Gew.-% Edukt.

### Beispiel 2

Analog Beispiel 1, aber mit 100 g eines mit Phosphorsäure (5 Gew.-%) dotierten Aluminiumoxids als Katalysator (Korngröße 0,1 - 0,3 mm), erhielt man bei 220°C und 6 Stunden Reaktionszeit 62 Gew.-% N-(2-Hydroxypropyl)-2,6-dimethylmorpholin neben 21 Gew.-% Edukt.

N-(2-Hydroxypropyl)-2,6-dimethylmorpholin ließ sich destillativ leicht von unumgesetztem Tris-(2-hydroxypropyl)-amin trennen, welches in die Reaktion rückführbar ist.

### Beispiel 3

In einem Reaktionsgefäß mit Tropftrichter und Destillationsaufsatz wurden 2,5 g mit Phosphorsäure (5 Gew.-%) dotierten Aluminiumoxids vorgelegt und auf 290°C aufgeheizt. Über den Tropftrichter wurde auf 60°C vorgewärmtes Tris-(2-hydroxypropyl)-amin kontinuierlich, unter geringem Gasstrom (Stickstoff), in das Reaktionsgefäß zugetropft. Über den Destillationsaufsatz wurden flüchtige Produkte abdestilliert und kondensiert. Das Kondensat bildete zwei Phasen, wobei die untere vorwiegend aus Wasser bestand. Innerhalb einer Stunde wurden 12 g Tris-(2-hydroxypropyl)-amin zugetropft. Nach 5, 30 und 60 Minuten wurde der Reaktionsaustrag analysiert. In der organischen Phase befanden sich demnach 68 Gew.-% (1,5 g), 63 Gew.-% (3,6 g) bzw. 65 Gew.-% (3,7 g) N-(2-Hydroxypropyl)-2,6-dimethylmorpholin und 3 Gew.-%, 3 Gew.-%, bzw. 5 Gew.-% Edukt. Die Gesamtausbeute an N-(2-Hydroxypropyl)-2,6-dimethylmorpholin im Destillat betrug 52 Mol.-%. Im Reaktionsgefäß verblieben ca. 2 g organische Phase, in der 15 Gew.-% N-(2-Hydroxypropyl)-2,6-dimethylmorpholin und 37 Gew.-% Edukt enthalten waren.

### Beispiel 4

3 g Tris-(2-hydroxypropyl)-amin und 1 g Tonsil wurden 45 Minuten auf 290°C in einem 50 ml Glasgefäß erhitzt. Nach Abkühlen und Abfiltrieren des Katalysators wurden GC-analytisch neben 12 Gew.-% Edukt 35 Gew.-% N-(2-Hydroxypropyl)-2,6-dimethylmorpholin gefunden.

### Beispiel 5

3 g Tris-(2-hydroxypropyl)-amin und 1 g mit Schwefelsäure dotiertes SnO₂ wurden analog Beispiel 5 1 Stunde lang auf 250°C erhitzt. Es fanden sich neben 50 Gew.-% Edukt 30 Gew.-% N-(2-Hydroxypropyl)-2,6-dimethylmorpholin.

### Beispiel 6

In einen 50 ml Metallautoklaven wurden 10 g (90 gew.-%ig) Tris-(2-hydroxypropyl)-amin und 2,7 g eines mit Wasserstoff aktivierten Katalysators (56 Gew.-% CuO, 44 Gew.-% Al₂O₃) gefüllt, 5 bar Wasserstoff aufgepreßt und 1 h auf 250°C erhitzt. Der Druck stieg wärend des Aufheizens auf 35 bar. Nach Abkühlen und Entspannen wurde vom Katalysator abfiltriert und das zweiphasige Filtrat (10 g) mit Ethanol homogenisiert und GC-analytisch untersucht. Neben 3 Gew.-% Edukt waren 67 Gew.-% N-(2-Hydroxypropyl)-2,6-dimethylmorpholin (Verhältnis des cis : trans Isomeren ca. 3 : 1) enthalten.

### Beispiel 7

Analog Beispiel 6 wurde ein Cu-Katalysator (10 Gew.-% CuO, 90 Gew.-% C, aktiviert) eingesetzt. Bei vollständigem Umsatz lagen im Austrag 87 Gew.-% N-(2-Hydroxypropyl)-2,6-dimethylmorpholin vor.

### Beispiel 8

Analog Beispiel 6 wurde ein überwiegend Cu-enthaltender Katalysator (37 Gew.-% CuO, 29 Gew.-% SiO₂, 14 Gew.-% MgO, Rest BaO, Cr₂O₃, ZnO und Wasser, aktiviert) eingesetzt. Neben 3 Gew.-% Edukt fanden sich im Austrag 77 Gew.-% N-(2-Hydroxypropyl)-2,6-dimethylmorpholin.

### Beispiel 9

Analog Beispiel 6 wurde ein Pd/Re-Katalysator (3 Gew.-% Re, 3 Gew.-% Pd auf Aktivkohle) eingesetzt. Neben 3 Gew.-% Edukt fanden sich im Austrag 60 Gew.-% N-(2-Hydroxypropyl)-2,6-dimethylmorpholin.

### Beispiel 10

In einen 2500 ml Metallautoklaven wurden 900 g (90 gew.-%ig) Tris-(2-hydroxypropyl)-amin und 160 g eines Cu-Katalysators (10 Gew.-% CuO, 90 Gew.-% C, aktiviert) gefüllt, 5 bar H₂ aufgepreßt und 2 h auf 250°C erhitzt. Der Reaktionsdruck stieg auf 35 bar. Nach Abkühlen wurde vom Katalysator abfiltriert und der Austrag destilliert. Es konnten neben 105 g Edukt 500 g (N-(3-Hydroxypropyl)-2,6-dimethylmorpholin rein isoliert werden.

## Patentansprüche

1. Verfahren zur Herstellung von N-(2-Hydroxypropyl)-2,6-dimethylmorpholin, dadurch gekennzeichnet, daß man Tris-(2-hydroxypropyl)-amin in Gegenwart
a) von sauren Katalysatoren oder
b) von Hydrierkatalysatoren cyclisiert.

2. Verfahren zur Herstellung von N-(2-Hydroxypropyl)-2,6-dimethylmorpholin, dadurch gekennzeichnet, daß man als saure Katalysatoren Oxide der III. oder IV. Nebengruppe oder der II. bis VI. Hauptgruppe des Periodensystems der Elemente oder Kombinationen derselben verwendet.

3. Verfahren zur Herstellung von N-(2-Hydroxypropyl)-2,6-dimethylmorpholin, dadurch gekennzeichnet, daß man als saure Katalysatoren saure Zeolithe, saure Schichtsilikate oder Heteropolysäuren verwendet.

4. Verfahren zur Herstellung von N-(2-Hydroxypropyl)-2,6-dimethylmorpholin, dadurch gekennzeichnet, daß man saure Katalysatoren, die mit Schwefelsäure oder Phosphorsäure behandelt wurden, einsetzt.

5. Verfahren zur Herstellung von N-(2-Hydroxypropyl)-2,6-dimethylmorpholin, dadurch gekennzeichnet, daß man die Umsetzung in Gegenwart von sauren Katalysatoren bei Temperaturen von 100 bis 400°C durchführt.

6. Verfahren zur Herstellung von N-(2-Hydroxypropyl)-2,6-dimethylmorpholin, dadurch gekennzeichnet, daß man als Hydrierkatalysatoren Elemente der I. oder VI. bis VIII. Nebengruppe des Periodensystems der Elemente verwendet.

7. Verfahren zur Herstellung von N-(2-Hydroxypropyl)-2,6-dimethylmorpholin, dadurch gekennzeichnet, daß man als Hydrierkatalysatoren Elemente der I. oder VI. bis VIII. Nebengruppe des Periodensystems der Elemente in Form ihrer Metalle, ihrer Oxide oder ihrer Sulfide verwendet.

8. Verfahren zur Herstellung von N-(2-Hydroxypropyl)-2,6-dimethylmorpholin, dadurch gekennzeichnet, daß die Hydrierkatalysatoren Kupfer enthalten.

9. Verfahren zur Herstellung von N-(2-Hydroxypropyl)-2,6-dimethylmorpholin, dadurch gekennzeichnet, daß man die Umsetzung in Gegenwart von Hydrierkatalysatoren bei Temperaturen von 150 bis 350°C durchführt.

10. Verfahren zur Herstellung von N-(2-Hydroxypropyl)-2,6-dimethylmorpholin, dadurch gekennzeichnet, daß man die Umsetzung in Gegenwart von Hydrierkatalysatoren und Wasserstoff durchführt.

## Claims

1. A process for preparing N-(2-hydroxypropyl)-2,6-dimethylmorpholine, which comprises cyclizing tris(2-hydroxypropyl)amine in the presence
a) of acidic catalysts or
b) of hydrogenation catalysts.

2. A process for preparing N-(2-hydroxypropyl)-2,6-dimethylmorpholine, which comprises using as acidic catalysts oxides of subgroup III or IV or of main group II to VI of the Periodic Table of the Elements or combinations thereof.

3. A process for preparing N-(2-hydroxypropyl)-2,6-dimethylmorpholine, which comprises using as acidic catalysts acidic zeolites, acidic sheet silicates or heteropolyacids.

4. A process for preparing N-(2-hydroxypropyl)-2,6-dimethylmorpholine, which comprises employing acidic catalysts which have been treated with sulfuric acid or phosphoric acid.

5. A process for preparing N-(2-hydroxypropyl)-2,6-dimethylmorpholine, which comprises carrying out the reaction in the presence of acidic catalysts at from 100 to 400°C.

6. A process for preparing N-(2-hydroxypropyl)-2,6-dimethylmorpholine, which comprises using as hydrogenation catalysts elements of subgroup I or VI to VIII of the Periodic Table of the Elements.

7. A process for preparing N-(2-hydroxypropyl)-2,6-dimethylmorpholine, which comprises using as hydrogenation catalysts elements of subgroup I or VI to VIII of the Periodic Table of the Elements in the form of their metals, their oxides or their sulfides.

8. A process for preparing N-(2-hydroxypropyl)-2,6-dimethylmorpholine, wherein the hydrogenation catalysts contain copper.

9. A process for preparing N-(2-hydroxypropyl)-2,6-dimethylmorpholine, wherein the reaction is carried out in the presence of hydrogenation catalysts at from 150 to 350°C.

10. A process for preparing N-(2-hydroxypropyl)-2,6-dimethylmorpholine, wherein the reaction is carried out in the presence of hydrogenation catalysts and hydrogen.

## Revendications

1. Procédé de préparation de la N-(2-hydroxypropyl)-2,6-diméthylmorpholine, caractérisé en ce que l'on procède à la cyclisation de la tris-(2-hydroxypropyl)-amine en présence
a) de catalyseurs acides, ou
b) de catalyseurs d'hydrogénation.

2. Procédé de préparation de la N-(2-hydroxypropyl)-2,6-diméthylmorpholine, caractérisé en ce que l'on utilise, à titre de catalyseurs acides, des oxydes des éléments du troisième ou du quatrième sous-groupe ou du second au sixième groupes principaux du système périodique des éléments, ou des combinaisons de ceux-ci.

3. Procédé de préparation de la N-(2-hydroxypropyl)-2,6-diméthylmorpholine, caractérisé en ce que l'on utilise, à titre de catalyseurs acides, des zéolites acides, des phyllosilicates, ou des hétéropolyacides.

4. Procédé de préparation de la N-(2-hydroxypropyl)-2,6-diméthylmorpholine, caractérisé en ce que l'on emploie des catalyseurs acides, qui ont été traités, par de l'acide sulfurique ou de l'acide phosphorique.

5. Procédé de préparation de la N-(2-hydroxypropyl)-2,6-diméthylmorpholine, caractérisé en ce que l'on entreprend la réaction en présence de catalyseurs acides à des températures de 100 à 400°C.

6. Procédé de préparation de la N-(2-hydroxypropyl)-2,6-diméthylmorpholine, caractérisé en ce que l'on utilise, à titre de catalyseurs d'hydrogénation des éléments des premier ou sixième à huitième sous-groupes du système périodique des éléments.

7. Procédé de préparation de la N-(2-hydroxypropyl)-2,6-diméthylmorpholine, caractérisé en ce que l'on utilise, à titre de catalyseurs d'hydrogénation des éléments des premier ou sixième à huitième sous-groupes du système périodique des éléments, sous la forme de leurs métaux, de leurs oxydes ou de leurs sulfures.

8. Procédé de préparation de la N-(2-hydroxypropyl)-2,6-diméthylmorpholine, caractérisé en ce que les catalyseurs d'hydrogénation contiennent du cuivre.

9. Procédé de préparation de la N-(2-hydroxypropyl)-2,6-diméthylmorpholine, caractérisé en ce que l'on entreprend la réaction en présence de catalyseurs d'hydrogénation à des températures de 150 à 350°C.

10. Procédé de préparation de la N-(2-hydroxypropyl)-2,6-diméthylmorpholine, caractérisé en ce que l'on entreprend la réaction en présence de catalyseurs d'hydrogénation et d'hydrogène.
